# EUROPÄISCHE PATENTSCHRIFT

(11) **EP 2 001 601 B1**
(45) Veröffentlichungstag und Bekanntmachung des Hinweises auf die Patenterteilung: **14.05.2014**
(21) Anmeldenummer: 07727330.8
(22) Anmeldetag: 26.03.2007
(51) Int. Cl.: B05B 11/00, A61M 15/00

(54) **VORRICHTUNG ZUR VERABREICHUNG VON PHARMAZEUTISCHEN ZUBEREITUNGEN**
DOSAGE AEROSOLS FOR THE ADMINISTRATION OF PHARMACEUTICAL PREPARATIONS
AÉROSOLS DE DOSAGE POUR L'ADMINISTRATION DE PRÉPARATIONS PHARMACEUTIQUES

(30) Priorität: 27.03.2006 DE 102006014433
(43) Veröffentlichungstag der Anmeldung: 17.12.2008
(73) Patentinhaber: Boehringer Ingelheim Pharma GmbH & Co. KG, 55216 Ingelheim am Rhein (DE)
(72) Erfinder: WACHTEL, Herbert, 55218 Ingelheim am Rhein (DE); HOELZ, Hubert, 55413 Oberheimbach (DE); ROHRSCHNEIDER, Marc, 58093 Hagen (DE)
(74) Vertreter: Simon, Elke Anna Maria
(86) Internationale Anmeldenummer: PCT/EP2007/052857
(87) Internationale Veröffentlichungsnummer: WO 2007/110403

(56) Entgegenhaltungen:
- WO-A-00/12162
- WO-A-96/03172
- WO-A-02/058771

## Beschreibung

Die Erfindung betrifft pharmazeutisch Wirkstoffe, Wirkstoffmischungen und -formulierungen zur Anwendung als Aerosol in treibgashaltigen [1.1.1.2-tetrafluorethan (HFC-134a) oder 1.1.1.2.3.3.3-heptafluorpropan (HFC-227)] Dosieraerosolen und die Dosieraerosole zur Applikation dieser Wirkstoffe, Wirkstoffmischungen und -formulierungen.

### GEBIET DER ERFINDUNG

Die Verwendung der Verabreichung von Aerosol-Formulierungen von Arzneimitteln mittels unter Druck stehenden, Dosis bemessenden Inhalatoren (MDIs, steht für "metereddose Inhalers") ist in der Therapie weit verbreitet, wie beispielsweise bei der Behandlung von Asthma und die Obstruktion der Luftwege verursachenden Krankheiten. Ein typisches MDI ist beispielsweise in der WO00/12162A1 in Fig. 1 abgebildet. Mit der oralen Verabreichung verglichen ergibt die Inhalation eine früher einsetzende Wirkung und gleichzeitig die Herabsetzung von systemischen Seitenwirkungen auf ein Minimum. Aerosol-Formulierungen können durch Inhalation durch den Mund oder topisch durch Auftragen auf die Nasenschleimhaut verabreicht werden.

Formulierungen zur Verabreichung von Aerosol über MDIs können aus Lösungen oder Suspensionen bestehen. Lösungsformulierungen bieten gegenüber den Suspensionsformulierungen den Vorteil, dass die Arzneimittel im Treibmittel vollständig gelöst sind und damit eine homogene Beschaffenheit aufzuweisen. Lösungs-Formulierungen vermeiden auch die mit Suspensionsformulierungen einhergehenden Probleme der physikalischen Instabilität und gewährleisten somit nachhaltige die Verabreichung gleicher Dosierungen. Darüber hinaus benötigen Lösungsdosieraerosole in der Regel nicht den Zusatz von Tensiden- Weiterhin gibt es so genannte "Suslutions", wie beispielsweise in US2004/0184994 offenbart.

Die Verabreichung von Aerosol-Lösungs-Formulierungen über MDIs ist von der Treibkraft des zu ihrer Herstellung verwendeten Treibmittelsystems abhängig.

Herkömmlicherweise enthielt das Treibmittel ein Gemisch von Chlorfluorkohlenwasserstoffen (CFCs, steht für" chlorofluorocarbons") zur Gewährleistung der gewünschten Eigenschaften Löslichkeit, Dampfdruck und Stabilität der Formulierung. Da jedoch mittlerweile feststeht, dass CFCs umweltschädigend sind, weil sie zur Verringerung der Ozonschicht der Erde beitragen, wird das in Aerosol-Inhalations-Formulierungen umweltschädigende CFC-Treibmittel durch umweltverträgliche teilfluorierte Kohlenwasserstoff-Treibmittel (HFC-Treibmittel) oder andere nichtchlorierte Treibmittel ersetzt.

Obwohl sehr viele Dosieraerosole auf dem Markt sind, ist deren Dosiergenauigkeit verbesserungsfähig.

Der vorliegenden Erfindung liegt die Aufgabe zugrunde, eine Vorrichtung mit einer erhöhten Dosiergenauigkeit für den Wirkstoff, die Wirkstoffmischung oder -formulierung bereitzustellen,

Die obige Aufgabe wird durch eine Vorrichtung gemäß Anspruch 1 gelöst. Vorteilhafte Weiterbildungen sind Gegenstand der Unteransprüche.

Der erste Aspekt der vorliegenden Erfindung liegt darin, dass das Mundrohr der Vorrichtung einen Kanal (8) besitzt, welcher an seiner dem Dosieraerosol abgewandten Seite kein oder fast kein Totvolumen besitzt.

Als "Totvolumen" wird der Raum definiert, der über die Düsenbohrung (9) hinausragt und somit nicht im direkten Strömungsverlauf liegt.

Weitere Vorteile, Merkmale, Eigenschaften und Aspekte der vorliegenden Erfindung ergeben sich aus der folgenden Beschreibung bevorzugter Ausführungsformen anhand der Zeichnung.

Es zeigt:

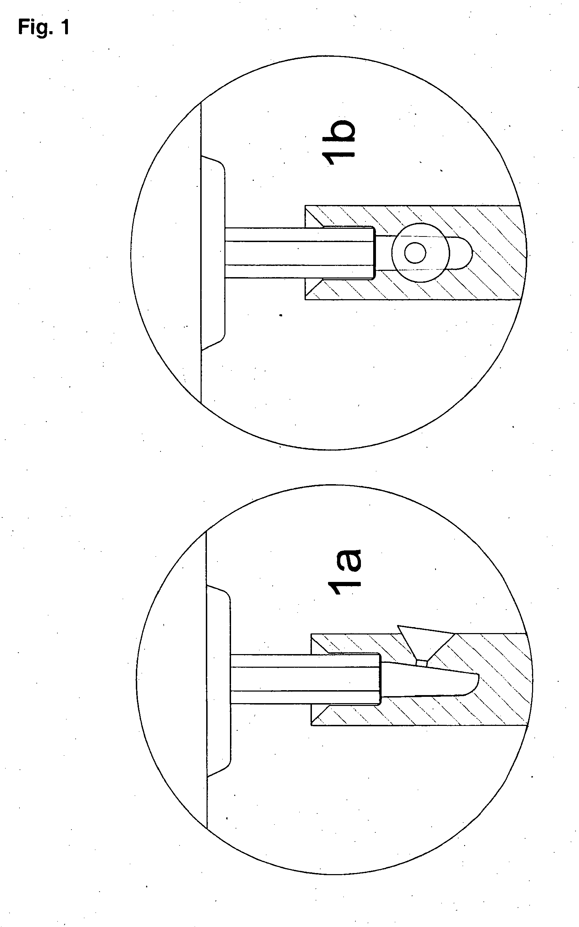

Fig. 1: Ein Mundrohr mit Totvolumen;

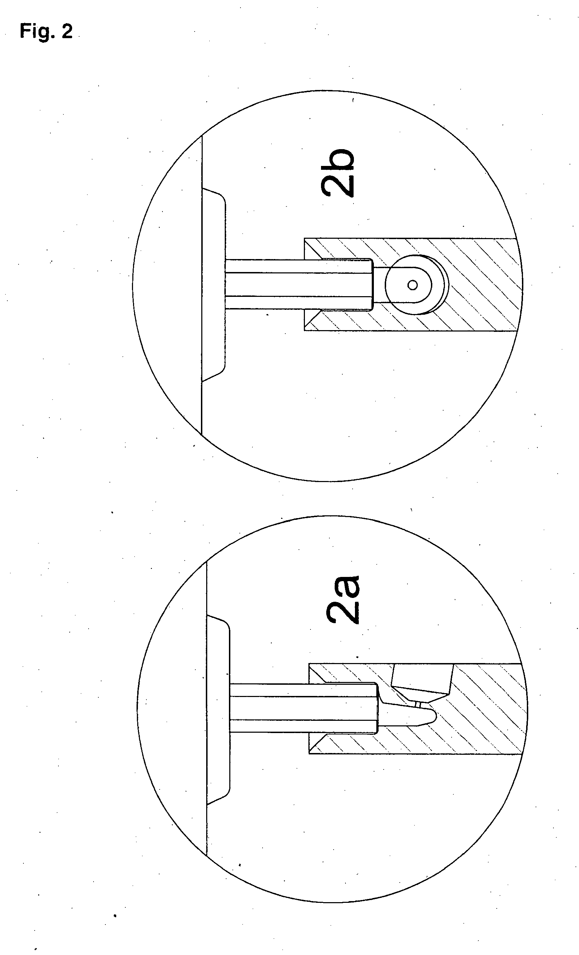

Fig. 2: Ein Mundrohr mit reduziertem Totvolumen;

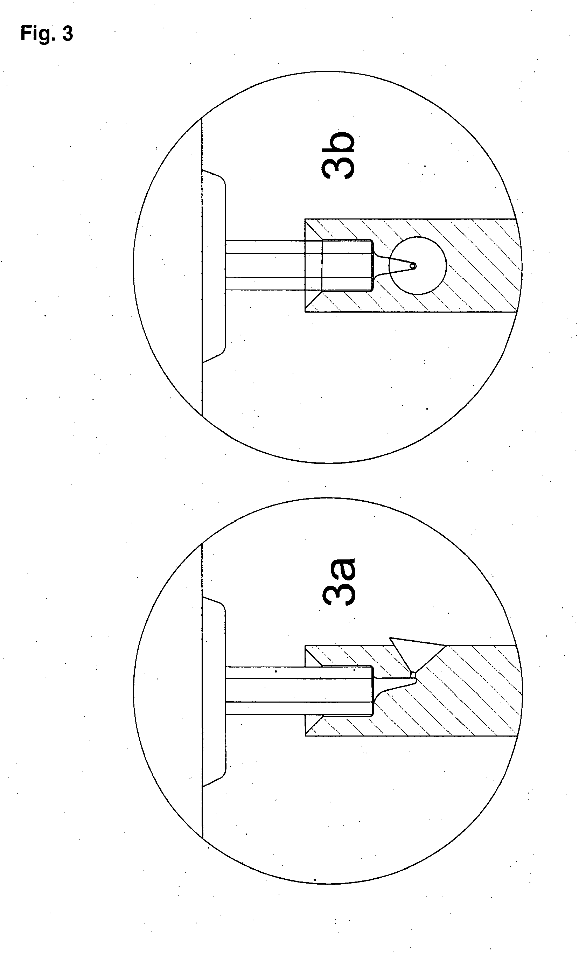

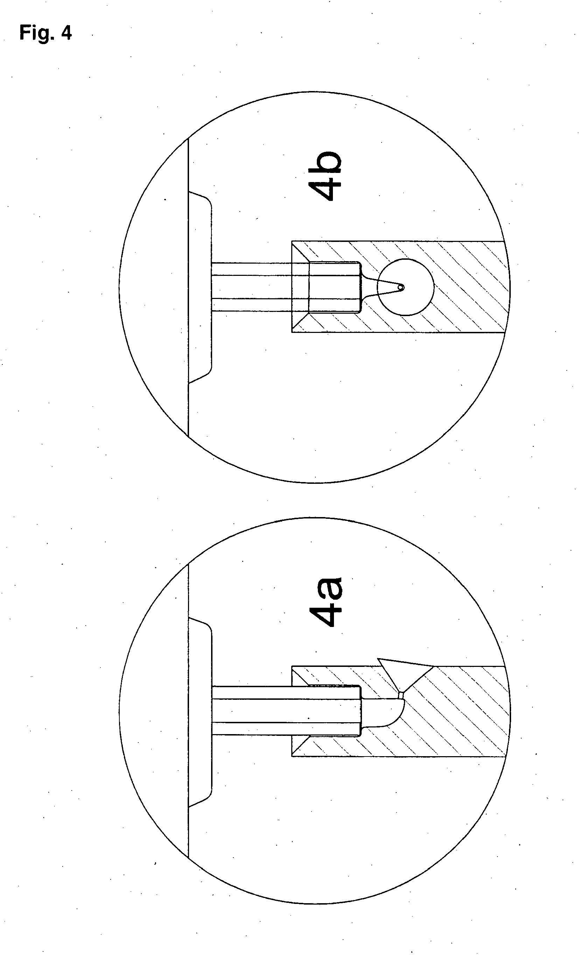

Fig. 3 und 4: Ein Mundrohr (das erfindungsgemäße Mundrohr jeweils) mit optimierter Reduktion des Totvolumens;
Fig. 5: Mundrohr wie Fig. 1a mit Bezugszeichen

Die mit "a" bezeichneten Figuren stellen jeweils die Ansicht eines Längsschnittes in der Seitenansicht dar.

Die mit "b" bezeichneten Figuren stellen jeweils die Ansicht eines Längsschnittes in der Aufsicht dar.

In den Figuren werden für gleiche oder ähnliche Teile die selben Bezugszeichen verwendet, wobei entsprechende oder vergleichbare Eigenschaften und Vorteile erreicht werden, auch wenn keine wiederholte Beschreibung erfolgt.

Das Mundrohr ist eine Komponente, die mehrere Funktionen erfüllt:

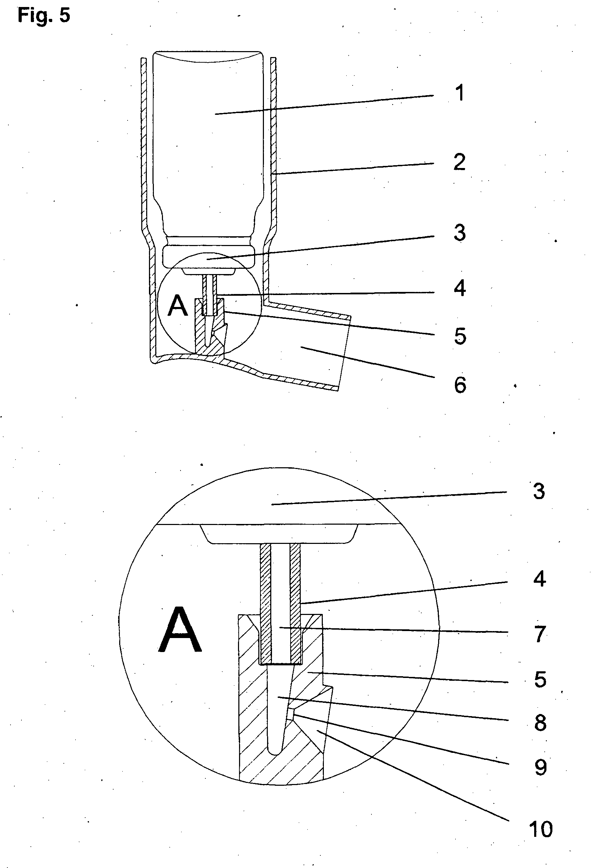

Das Mundrohr gemäß Figur 5 besteht funktional aus einem Schaft (2), einer Ventilstammaufnahme (5), einer Düsenbohrung (9) sowie dem Mundstück (6). Das Mundrohr nimmt das Dosieraerosol so auf, dass das der Mundrohrschaft: (2) das Dosieraerosol (1) umhüllt und die Ventilstammaufnahme (5) des Mundrohrs den Ventilstamm (4) des Dosierventils (3) dichtend umschließt. Der Mundrohrschaft (2) hat die Funktion ein für das Ventil (3) schädliches Verkanten von dem Ventilstamm (4) zu den übrigen Ventilkomponenten (3) sicher zu verhindern indem er den Bewegungsspielraum des Dosieraerosols einschränkt. Des Weiteren bildet die Ventilstammaufnahme (5) ein Widerlager das den Eintauchweg des Ventilstamms (4) in die Stammaufnahme des Mundrohrs begrenzt.

Die Ventilstammaufnahme besitzt einen Kanal (8) auf den in einem stumpfen Winkel zu seiner Senkrechten Achse die eigentliche Düsenbohrung (9) stößt. Die Düsenbohrung hat einen Durchmesser von 0,2 - 0,6 mm, vorzugsweise zwischen 0,5 - 0,6 mm für Suspensionsformulierungen und Suslutions, sowie zwischen 0,2 - 0,27 mm für Lösungsformulierungen.

Dadurch kann die unter Druck verflüssigte Wirkstofformulierung von der senkrecht nach unten angeordneten Öffnung des Ventilstamms (7) um einen stumpfen Winkel herum umgelenkt und durch die Düsenbohrung (9) nach außen gelangen. Die Sprühwolke wird schließlich am Übergang der Düsenbohrung (9) zur Düsenöffnung (10) generiert,

Die in der Erfindung beschriebene Veränderung des Mundrohrs wird durch eine wesentlich genauere Positionierung der Werkzeugteile Düsennadel und Ventilstammaufnahmekern zueinander im herkömmlichen Spritzgussverfahren realisiert.

Beispxelhafte Abmessungen von Ventilstämmen:

| Ventil/Hersteller | Aussendurchmesser des Ventilstamms | Innendurchmesser des Ventilstamms an der Öffnung |
|---|---|---|
| Bespak, BK357 | 3,17 ± 0,01mm | 2,12 ± 0,03mm |
| Valois, DF31/50RCU | 3,19 ± 0,01mm | 1,48 ± 0,02mm |
| 3M, Neotechnic | 2,76 ± 0,01mm | 1,68 ± 0,04mm |

Der zweite Aspekt der vorliegenden Erfindung liegt darin, dass der Kanal (8) über eine Düsenbohrung (9) mit einer Düsenöffnung verbunden ist, die trichterförmig ausgebildet ist. Aus der Beschreibung wird ersichtlich, dass die erfindungsgemäße Vorrichtung geeignet ist, geeigneten Wirkstoff, jede geeignete Wirkstoffmischung oder -formulierung als Einzeldosierungen zu applizieren.

Als pharmazeutisch wirksame Wirkstoffe, Wirkstoffmischungen oder -formulierungen (pharmazeutische Zubereitung) werden alle inhalierbaren Verbindungen eingesetzt, wie z.B auch inhaherbare Makromoleküle, wie in EP 1 003 478 offenbart. Vorzugsweise werden Wirkstoffe, Wirkstoffmischungen oder -formulierungen zur Behandlung von Atemwegserkrankungen eingesetzt, die im inhalativen Bereich Verwendung finden.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoethanne, Isoprenaline, Levosalbutamol, Mabuterol, Meluadnne, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl }-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamibo]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylammo}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylammo}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylammo}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylammo]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chlondsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlond, Bromid, lodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 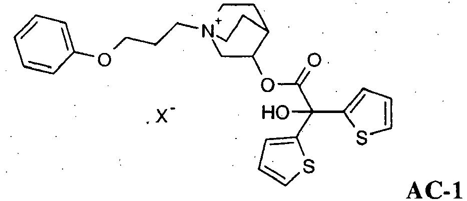 worin X ⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 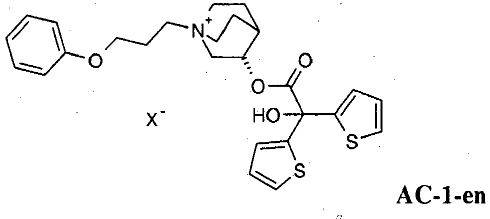 enthalten, worin X - die vorstehend genannten Bedeutungen aufweisen kann. Weiterhin bevorzugte Anticholinergika sind ausgewählt aus den Salzen der Formel **AC-2** 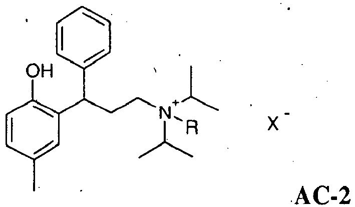 worin R entweder Methyl oder Ethyl bedeuten und worin X - die vorstehend genannte Bedeutungen aufweisen kann. In einer alternativen Ausführungsform kann die Verbindung der Formel **AC-2** auch in Form der freien Base **AC-2-base** vorliegen.

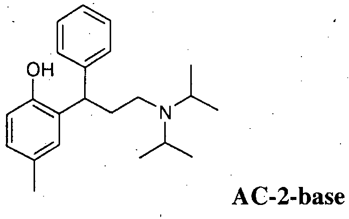

Weiterhin genannte Verbindungen sind:
- 2,2-Diphenylproponsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsaurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsauretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsaurescopinester-Methobromid
- 9-Methyl-xanthen-9-carbonsäuretropenolester-Methobromid
- 9-Methyl-xanthen-9-carbonsaurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsaure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester gegebenenfalls in Form ihrer Racemate, Enantiomere oder Diastereomere und gegebenenfalls in Form ihrer Salze und Derivate, ihrer Solvate und/oder Hydrate Jede Bezugnahme auf Steroide schließt eine Bezugnahme auf deren gegebenenfalls existierende Salze oder Denvate, Hydrate oder Solvate mit ein. Beispiele möglicher Salze und Derivate der Steroide können sein Alkalisalze, wie beispielsweise Natnum- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Anflo (Cilomilast), Tofimilast, Pumafentnn, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, C1-1018, CDC-801, CDC-3052, D-22888, YM-58997, Z-15370 und
- N-(3,5-Dichloro-1-oxo-pyndin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyndin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrodin-2-yliden]acetat
- 9-Cyclopentyl-5,6-dihydro-7-ethyl-3-(2-thienyl)-9*H*-pyrazolo[3,4-c]-1,2,4-tnazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate Erfindungsgemäß bevorzugt sind die Säureadditionssalze der PDE4-Inhibitoren ausgewahlt aus der Gruppe bestehend aus Hydrochlond, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)etheny)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydromtrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Denvaten zu deren Bildung die LTD4-Antagonisten gegebenenfalls in der Lage sind, werden beispielsweise verstanden: Alkalisalze, wie beispielsweise Natnum- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]ammo}-7-[(R)-(tetrahydrofuran-2-yl)methox y]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonylethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxychinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-pipendin-4-yloxy)-7-methoxychinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxychinazolin
- 4-[(3-Chlor-4-fluor-phenyl)amino]-6-(tetrahydropyran-4-yloxy)-7-(2-methoxyethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-l-yloxy}-7-methoxy-chinazolin
- 4-[(3-Chlor-4-fluor-phenyl)amino]-6-(tetrahydropyran-4-yloxy)-7-(2-acetylaminoethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methylamino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methylamino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxychinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methylamino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-pipendin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-pipendin-4-yloxy)-7-methoxychinazolin
- 4-[(3-Chlor-4-fluor-phenyl)amino]-6-(1-methyl-piperidin-4-yloxy)-7(2-methoxyethoxy)-chinazolin
- 4-[(3-Chlor-4-fluor-phenyl)amino]-6-(1-isopropyloxycarbonyl-pipendin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxychinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethylamino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlond, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccmat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocnptin, Cabergolin, Alpha-Dihydroergocryptin, Lisund, Pergolid, Pramipexol, Oxindol, Ropinirol, Talipexol, Tergund und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfingdungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadm, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlond, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromolekule verwendet werden, wie in EP 1 003 478 offenbart.

Weiterhin kann die Verbindung aus der Gruppe der Denvate von Mutterkornalkaloiden, der Tnptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Fur die Inhalation kommen Arzneimittel, Arzneimittelformuherungen und -mischungen mit den o.g. Wirkstoffen in Betracht, sowie deren Salze, Ester sowie die Kombination dieser Wirkstoffe, Salze und Ester.

Die pharmazeutische Zubereitung kann zusätzlich handelsübliche Suspensionshilfsstoffe, Tenside und/oder Stabilisatoren enthalten.

## Patentansprüche

1. Vorrichtung zur Bereitstellung von pharmazeutischen Wirkstoffen, Wirkstoffmischungen und -formulierungen umfassend
- ein Gefäß, das mit einer pharmazeutischen Zubereitung gefüllt ist, enthaltend einen aktiven Wirkstoff, eine Wirkstoffmischung oder -formulierung in druckverflüssigtem 1.1.1.2-tetrafluorethan (HFC-134a) oder 1.1.1.2.3.3.3-heptafluorpropan (HFC-227) oder ein Gemisch aus HfC-134a und HFC-227 und
- ein Ventil, das angeordnet ist, um Einzeldosierungen einer pharmazeutischen Zubereitung außerhalb der Vorrichtung abzugeben, und einen Ventilstamm (4) aufweist, und
- ein Mundrohr bestehend aus einem Mundstück (6), einem Schaft (2), einer Ventilstammaufnahme (5), einer Düsenbohrung (9), wobei die Ventilstammaufnahme (5) einen Kanal (8) besitzt, auf welchen in einem stumpfen Winkel zu seiner senkrechten Achse die eigentliche Düsenbohrung (9) stößt, wodurch das unter Druck verflüssigte Arzneimittel, die Arzneimittelmischung oder -formulierung von der senkrecht nach unten angeordneten Öffnung (7) des Ventilstamms (4) umgelenkt und durch die Düsenbohrung (9) nach außen gelangt,
**dadurch gekennzeichnet, dass** der Querschnitt der Öffnung des Kanals (8) geringfügig größer als der Querschnitt der Öffnung (7) des Ventilstamms (4) ist und sich der Kanal (8) von seiner Öffnung bis zu einem Ende erstreckt, an dem die Düsenbohrung (9) angeordnet ist, wobei sich der Kanal (8) derart in Richtung der Düsenbohrung (9) verjüngt, dass die Breite des Kanals (8) am Ende des Kanals (8) in der Ebene der Düsenbohrung (9) in etwa dem Durchmesser der Düsenbohrung (9) an dieser Stelle entspricht, so dass der Kanal (8) kein oder fast kein Totvolumen besitzt.

2. Vorrichtung nach Anspruch 1, **dadurch gekennzeichnet, dass** die pharmazeutische Zubereitung zusätzlich Suspensionshilfsstoffe, Tenside und/oder Stabilisatoren enthält.

3. Vorrichtung nach Anspruch 1 oder 2, **dadurch gekennzeichnet, dass** der Kanal (8) über eine Düsenbohrung (9) mit einer Düsenöffnung (10) verbunden ist, die trichterförmig ausgebildet ist.

4. Vorrichtung nach einem oder mehreren der obigen Ansprüche, **dadurch gekennzeichnet, dass** die Düsenbohrung (9) einen Durchmesser von 0,2 - 0,6 mm hat.

5. Vorrichtung nach einem oder mehreren der obigen Ansprüche **dadurch gekennzeichnet, dass** das Ventil ein Dosierventil (3) ist, dass die Ventilstammaufnahme (5) des Mundrohrs den Ventilstamm (4) des Dosierventils (3) dichtend umschließt und dass beim Austritt von unter Druck verflüssigtem Arzneimittel, Arzneimittelmischung oder -formulierung durch die Düsenbohrung (9) die Sprühwolke am Übergang der Düsenbohrung (9) zur Düsenöffnung (10) generiert wird.

## Claims

1. Device for supplying pharmaceutical active substances, active substance mixtures and formulations comprising
- a container filled with a pharmaceutical preparation, containing an active substance, an active substance mixture or formulation in pressure-liquefied 1.1.1.2-tetrafluoroethane (HFC-134a) or 1.1.1.2.3.3.3-heptafluoropropane (HFC-227) or a mixture of HFC-134a and HFC-227 and
- a valve which is arranged so as to deliver single doses of a pharmaceutical preparation outside the device and comprises a valve stem (4), and
- a mouthpiece tube consisting of a mouthpiece (6), a shaft (2), a valve stem receptacle (5), a nozzle bore (9), the valve stem receptacle (5) comprising a channel (8) on which the actual nozzle bore (9) abuts at an obtuse angle to its perpendicular axis, as a result of which the medicament, the medicament mixture or formulation liquefied under pressure is deflected from the perpendicularly downwardly arranged opening (7) of the valve stem (4) and passes outwards through the nozzle bore (9),
**characterised in that** the cross-section of the opening of the channel (8) is slightly larger than the cross-section of the opening (7) of the valve stem (4) and the channel (8) extends from its opening to an end at which the nozzle bore (9) is arranged, the channel (8) tapering in the direction of the nozzle bore (9) such that the width of the channel (8) at the end of the channel (8) in the plane of the nozzle bore (9) roughly corresponds to the diameter of the nozzle bore (9) at this point, so that the channel (8) has no or almost no dead volume.

2. Device according to claim 1, **characterised in that** the pharmaceutical preparation additionally contains suspension adjuvants, surfactants and/or stabilisers.

3. Device according to claim 1 or 2, **characterised in that** the channel (8) is connected, via a nozzle bore (9), to a nozzle opening (10) which is of funnel-shaped construction.

4. Device according to one or more of the above claims, **characterised in that** the nozzle bore (9) has a diameter of 0.2 - 0.6 mm.

5. Device according to one or more of the above claims, **characterised in that** the valve is a metering valve (3), **in that** the valve stem receptacle (5) of the mouthpiece tube sealingly encloses the valve stem (4) of the metering valve (3) and **in that** as the pressure liquefied medicament, medicament mixture or formulation exits through the nozzle bore (9) the spray mist is generated at the transition from the nozzle bore (9) to the nozzle opening (10).

## Revendications

1. Dispositif de préparation de substances actives pharmaceutiques, de mélanges et de formulations de substances actives, comprenant
- un récipient qui est rempli avec une préparation pharmaceutique contenant une substance active, un mélange ou une formulation de substance active dans du 1,1,1,2-tétrafluoroéthane (HFC-134a) ou dans du 1,1,1,2,3,3,3,-heptafluoropropane (HFC-227) ou un mélange de HFC-134a et de HFC-227 liquéfiés sous pression et
- une soupape qui est disposée pour distribuer des dosages individuels d'une préparation pharmaceutique hors du dispositif et présente un corps de soupape (4),
- un tube buccal consistant en un embout buccal (6), une tige (2), une ouverture du corps de soupape (5), un alésage de buse (9), dans lequel l'ouverture de corps de soupape (5) possède un canal (8) sur lequel se trouve, dans un angle obtus par rapport à son axe vertical, l'alésage de buse (9) proprement dit, par lequel, le médicament, le mélange ou
la formulation de médicament liquéfiés sous pression sont dérivés de l'ouverture (7), disposée verticalement vers le bas, du corps de soupape (4) et parviennent à l'extérieur par l'alésage de buse (9),
**caractérisé en ce que** la section de l'ouverture du canal (8) est légèrement plus grande que la section de l'ouverture (7) du corps de soupape (4) et le canal (8) s'étend de son ouverture jusqu'à une extrémité sur laquelle l'alésage de buse (9) est disposé, dans lequel le canal (8) s'affine en direction de l'alésage de buse (9) de telle manière que la largeur du canal (8) à la fin du canal (8) correspond dans le plan de l'alésage de buse (9) à peu près au diamètre de l'alésage de buse (9) à cet endroit de sorte que le canal (8) ne possède pas ou pratiquement pas de volume mort.

2. Dispositif selon la revendication 1, **caractérisé en ce que** la préparation pharmaceutique contient en outre des adjuvants de suspension, des tensioactifs et/ou des stabilisants.

3. Dispositif selon la revendication 1 ou 2, **caractérisé en ce que** le canal (8) est relié par un alésage de buse (9) à une ouverture de buse (10) qui est en forme d'entonnoir.

4. Dispositif selon une ou plusieurs des revendications ci-dessus, **caractérisé en ce que** l'alésage de buse (9) a un diamètre de 0,2 à 0,6 mm.

5. Dispositif selon une ou plusieurs des revendications ci-dessus, **caractérisé en ce que** la soupape est une soupape de dosage (3), **en ce que** l'ouverture du corps de soupape (5) de l'embout buccal entoure de façon étanche le corps de soupape (4) de la soupape de dosage (3) et **en ce que** lors de la sortie du médicament, du mélange ou de la formulation de médicament sous pression par l'alésage de buse (9), le nuage de pulvérisation est généré lors du passage de l'alésage de buse (9) vers l'ouverture de buse (10).
